# EUROPÄISCHE PATENTSCHRIFT

(11) **EP 3 849 484 B1**
(45) Veröffentlichungstag und Bekanntmachung des Hinweises auf die Patenterteilung: **29.05.2024**
(21) Anmeldenummer: 19765670.5
(22) Anmeldetag: 30.08.2019
(51) Int. Cl.: A61F 13/20

(54) **VORRICHTUNG UND VERFAHREN ZUM BESTÜCKEN VON TAMPON-APPLIKATOREN MIT TAMPONS**
DEVICE AND METHOD FOR LOADING TAMPON APPLICATORS WITH TAMPONS
DISPOSITIF ET PROCÉDÉ D'ÉQUIPEMENT EN TAMPONS D'APPLICATEURS DE TAMPON

(30) Priorität: 10.09.2018 CH 10692018
(43) Veröffentlichungstag der Anmeldung: 21.07.2021
(73) Patentinhaber: Ruggli Projects AG, 6332 Hagendorn (CH)
(72) Erfinder: SCHULER, Samuel, 4051 Basel (CH); BAUMGARTNER, Patrick, 5306 Tegerfelden (CH)
(74) Vertreter: IPrime Rentsch Kaelin AG
(86) Internationale Anmeldenummer: PCT/EP2019/073210
(87) Internationale Veröffentlichungsnummer: WO 2020/052999

(56) Entgegenhaltungen:
- EP-A1- 2 335 666
- US-A- 2 624 078
- US-A- 4 321 993

## Beschreibung

Die vorliegende Erfindung betrifft eine eine Haltekassette zur Aufnahme eines Tampon-Applikators gemäss Ansprüche 1-3.

### Technologischer Hintergrund

Tampon-Applikatoren sind meistens aus Kunststoff bestehende, röhrenförmige Einführhilfen für Tampons. In ihrer einfachsten Bauweise weisen sie einen Hülsenkörper mit einer distalen Öffnung auf, durch welche der Tampon nach dem Platzieren in die Körperöffnung entlassen wird, auf. Das Ausstossen des Tampons geschieht mittels eines proximal angelegten Stössels. Das Einschieben des Stössels in den Hülsenkörper drückt den darin enthaltenen Tampon durch die besagte distale Öffnung. Ausgereiftere Formen verfügen über ein abgerundetes distales Kopfende, welches mit Einschnitten versehen ist, die sich beim Herausdrücken des Tampons blütenartig öffnen. Auch Griffmulden oder gerippte Abschnitte gehören inzwischen zu den standardmässigen Ausgestaltungen, um die Anwendbarkeit zu erleichtern. Oft handelt es sich beim Stössel um ein Röhrchen, durch welches ein Rückholfaden des Tampons geführt ist. In der Anwendung wird dieser in der Hand behalten, sodass er sicher platziert werden kann.

Bei der Bestückung von Tampon-Applikatoren muss ein Tampon in einen Tampon-Applikator überführt werden. Dies kann auf verschiedene Weise geschehen. Einige Verfahren zur Bestückung von Tampon-Applikatoren basieren auf dem Prinzip eines Vorderladers, bei dem der Tampon durch die spätere distale Austrittsöffnung in den Hohlraum geführt wird. Es gibt auch Verfahren, bei denen die Tampons durch die proximale Öffnung bestückt werden. Bei Letzterem muss allerdings der Stössel, der im Betrieb dazu dient, den Tampon aus der distalen Öffnung zu stossen, entweder später angebracht werden, oder er muss so ausgelegt sein, dass der Tampon an ihm vorbei oder durch ihn durch in einen Tamponkörper gebracht werden kann.

Die vorliegende Erfindung ist zum Beispiel für Tampons mit Applikatoren geeignet, wie sie in der EP 3'016'623 A1 oder der EP 2'398'437 B1 gezeigt sind.

Die meisten Vorrichtungen und Verfahren zur Bestückung von Tampon-Applikatoren mit Tampons arbeiten intermittierend. Das heisst es wird stets ein Tampon-Applikator bereitgestellt und in einem Bestückungsschritt bestückt. Dazu hält die Vorrichtung den Tampon-Applikator in einer entsprechenden Ausrichtung für einen gewissen Zeitraum. Bei schnell getakteten Vorrichtungen kann dieser Zeitraum sehr gering sein. Nichtsdestotrotz ist die intermittierende und nicht kontinuierliche Bestückung ein limitierender Faktor in der Produktionsgeschwindigkeit von Tampons mit Tampon-Applikatoren. Zudem führt das schnell getaktete Stop-and-go-System einer intermittierenden Bestückung zu einem erhöhten Materialverschleiss. EP2335666 beschreibt eine Vorrichtung zur Bestücken von Tampon Applikatoren.

Es besteht somit ein Bedarf an Vorrichtungen und Verfahren zur Bestückung von Tampon-Applikatoren mit Tampons, welche eine hohe Prozessgeschwindigkeit ermöglichen.

### Darstellung der Erfindung

Es ist somit eine Aufgabe der vorliegenden Beschreibung, mindestens einen Nachteil des Bekannten zu überwinden. Insbesondere soll eine Vorrichtung zur Bestückung von Tampon-Applikatoren mit Tampons, sowie ein entsprechendes Verfahren und Führungseinheiten zum Transport von Tampon-Applikatoren auf einer Führungsanordnung bereitgestellt werden, welche den hohen Anforderungen einer kontinuierlichen Bestückung von Tampon-Applikatoren entgegenkommt. Insbesondere soll eine entsprechende Vorrichtung und ein Verfahren bereitgestellt werden, welches nicht nur kontinuierlich arbeiten kann, sondern auch skalierbar ist.

Diese Aufgabe wurde mit einer Vorrichtung zur Bestückung von Tampon-Applikatoren mit Tampons, einem entsprechenden Verfahren und einer Führungseinheit gemäss kennzeichnenden Teilen der unabhängigen Ansprüche gelöst.

Ein Aspekt der vorliegenden Beschreibung betrifft eine Vorrichtung zur Bestückung von Tampon-Applikatoren mit Tampons. Die Vorrichtung umfasst eine umlaufende Führungsanordnung mit einer Eingangsstelle. An dieser Eingangsstelle werden die Tampon-Applikatoren in die Vorrichtung eingespeist. Die umlaufende Führungsanordnung verfügt weiterhin über eine Ausgangsstelle, an der bestückte Tampon-Applikatoren ausgeschieden werden. Die Vorrichtung umfasst weiter eine Bestückungseinheit, welche zwischen der Eingangsstelle und der Ausgangsstelle angeordnet ist. Sie ist ausgestaltet, um Tampon-Applikatoren mit Tampons zu bestücken. Zum Transport der Tampon-Applikatoren auf der Führungsanordnung ist eine Mehrzahl von auf der Führungsanordnung geführter Führungseinheiten vorgesehen.

Die Vorrichtung ermöglicht eine kontinuierliche Zufuhr von Tampon-Applikatoren an eine Bestückungseinheit, welche zudem durch eine Anzahl an Führungseinheiten skalierbar ist. So kann z.B. die Anzahl der Führungseinheiten so ausgestaltet sein, dass sie ein entsprechendes Optimum zwischen der Anzahl der Tampon-Applikatoren, welche an der Eingangsstelle bereitgestellt werden, und der Bearbeitungsgeschwindigkeit der Bestückungseinheit gewählt werden kann.

In einer besonderen Ausführungsform ist die Bestückungseinheit trommelförmig ausgebildet und weist radial angeordnete Tampon-Aufnahmen auf, durch welche der Tampon in den Tampon-Applikator überführt wird und das Bestücken stattfindet.

In einer besonderen Ausführungsform umfasst die Führungsanordnung mindestens eine Schiene. Diese Schiene kann so ausgestaltet sein, dass die auf der Führungsanordnung geführten Führungseinheiten durch die Schiene auf der Führungsanordnung bewegbar gelagert sind.

In einer besonders einfachen Ausführungsform dieser Ausgestaltung umfasst die Führungsanordnung eine als T-Profil ausgestaltete Schiene, durch die entsprechende Gegenstücke der Führungseinheiten auf der Führungsanordnung geführt sind.

Im Sinne der vorliegenden Erfindung kann ein Eingespeistwerden ein passiver Vorgang sein, bei dem z.B. ein Tampon-Applikator durch ein Drittmittel in die Vorrichtung, insbesondere eine Führungseinheit der Vorrichtung überführt wird, oder es kann sich um einen aktiven Prozess handeln, bei dem die Vorrichtung, insbesondere die Führungseinheit mindestens ein Mitnahmemittel aufweist, um den Tampon-Applikator in die Vorrichtung aufzunehmen.

Analog kann es sich beim Ausgeschiedenwerden um einen passiven Prozess handeln, bei dem ein weiteres Drittelement den Tampon-Applikator aus der Vorrichtung, insbesondere einer Führungseinheit entfernt, oder aber es kann sich um einen aktiven Prozess handeln, bei dem ein Tampon-Applikator aktiv aus der Vorrichtung, insbesondere einer Führungseinheit ausgeschieden wird.

In einer besonderen Ausführungsform kann sowohl das Eingespeistwerden als auch das Ausgeschiedenwerden mittels eines Stössels bewerkstelligt werden, der den entsprechenden Tampon-Applikator aus, respektive in die Vorrichtung, insbesondere Führungseinheit ausstösst, oder einführt.

In einer besonderen Ausführungsform ist die Bestückungseinheit der Eingangsstelle in einer Förderrichtung nachgelagert. Besonders bevorzugt ist die Bestückungseinheit so angeordnet, dass sie einen Wirkungskreis aufweist, der sich mit mindestens einem Bereich der umlaufenden Führungsanordnung überlappt.

In einer besonderen Ausführungsform sind die Führungseinheiten als Wagen ausgestaltet, welche entlang der Führungsanordnung verschiebbar gelagert sind. Die Wagen können über Rollen mit einer Schiene der Führungsanordnung gelagert sein.

In einer besonderen Ausführungsform ist jede Führungseinheit zum Transport eines einzelnen Tampon-Applikators ausgestaltet. Dazu kann die Führungseinheit z.B. eine Tampon-Applikator-Aufnahme aufweisen, welche zur Aufnahme eines Tampon-Applikators ausgestaltet ist.

Durch den Transport der Tampon-Applikatoren auf der Führungsanordnung mittels als Wagen ausgestalteter Führungseinheiten kann die Vorrichtung optimal an die Bearbeitungsgeschwindigkeiten der Bearbeitungsstationen angepasst werden. So können zum Beispiel eine oder mehrere Pufferbereiche vorgesehen werden, die eine bestimmte Anzahl an Führungseinheiten sammeln, um sie dann stossweise als abgeteilte Menge der nächsten Bearbeitung zuzuführen.

In einer besonderen Ausführungsform ist der Transport auf der Führungsanordnung umlaufend. Die Führungsanordnung kann einen geschlossenen Kreislauf beschreiben, mit einer Eingangsstelle, an der die Tampon-Applikatoren unbestückt in die Vorrichtung eingespeist werden, und einer Ausgangsstelle, an der die Tampon-Applikatoren bestückt die Vorrichtung verlassen. Die Führungseinheiten in dieser Ausführungsform sind so angeordnet, dass sie auf der Führungsanordnung umlaufend verschiebbar gelagert sind. Sie übernehmen an der Eingangsstelle einen Tampon-Applikator und führen ihn an die Bestückungseinheit, wo der Tampon-Applikator mit einem Tampon bestückt wird, und letztlich zur Ausgangsstelle, wo die bestückten Tampon-Applikatoren ausgeschieden werden.

In einer besonderen Ausführungsform beschreibt die Führungsanordnung eine geschlossene Bahn.

In einer weiteren besonderen Ausführungsform umfasst die Führungsanordnung eine Schiene, welche eine geschlossene Bahn beschreibt. Die Führungsanordnung kann lineare Strecken aufweisen, welche in Kurven münden.

In einer besonderen Ausführungsform beschreibt die Führungsanordnung einen im Wesentlichen ovalen Förderweg. Alternativ kann die gesamte Führungsanordnung kreisförmig ausgestaltet sein.

In einer besonderen Ausführungsform umfasst die Führungsanordnung mindestens einen Bearbeitungsraum mit mindestens einer Bearbeitungseinheit.

In einer weiteren besonderen Ausführungsform ist dieser Bearbeitungsraum entlang eines linearen Abschnittes der Führungsanordnung angelegt. Der Bearbeitungsraum kann so ausgestaltet sein, dass er eine Reihe von hintereinander angeordneter Bearbeitungseinheiten umfasst. Die Bearbeitungseinheiten können so hintereinander angeordnet sein, dass sie sukzessive Bearbeitungsschritte an einem Werkstück vorzunehmen vermögen. Besonders bevorzugt werden sukzessive Bearbeitungsschritte am Tampon-Applikator vorgenommen.

In einer besonderen Ausführungsform umfasst der Bearbeitungsraum mindestens ein Heizelement, mindestens ein Formungselement und mindestens ein Kühlelement.

Bevorzugt ist das Heizelement ausgestaltet, um den Tampon-Applikator zu erwärmen. Üblicherweise sind Tampon-Applikatoren aus einem thermoplastischen Kunststoff gefertigt. Besonders bevorzugt ist das Heizelement also so ausgestaltet, dass er eine Aufweichung des thermoplastischen Kunststoffs des Tampon-Applikators erreicht, ohne dass dieser dabei zu schmelzen beginnt. Die dazu erforderliche Temperatur kann abhängig vom gewählten Material des Tampon-Applikators sein. Bevorzugt ist das Heizelement so ausgestaltet, dass es eine Temperatur von ca. 100 °C zu erreichen vermag +/- 10 bis 20 °C. Das Heizelement kann auch so ausgestaltet sein, dass gezielt nur umzuformende Elemente erwärmt werden, insbesondere das distale Ende des Tampon-Applikators.

In einer weiteren Bearbeitungseinheit, welche als Formungselement ausgestaltet ist, kann z.B. ein Element des Tampon-Applikators in eine neue Form überführt werden. In der Praxis bedeutet das meistens, dass ein Tampon-distales Ende umgeformt wird. Meistens ist das distale Ende als blütenförmige Öffnung ausgestaltet, welche bei der Anwendung den Tampon durch Druck des Stössels am proximalen Ende auszustossen vermag. In der Bestückung ist dieses distale Ende blütenförmig offen, damit der Tampon durch das distale Ende in den Tampon-Applikator eingeführt werden kann. Um den Tampon-Applikator in seine endgültige Form umzuformen, kann eine entsprechende Form im Formungselement vorgesehen sein, welche dem Tampon-Kopf formweise im Wesentlichen entspricht und den durch das Heizelement vorgewärmten Tampon-Applikator derart mit Druck beaufschlagt, dass dieser am distalen Ende seine geschlossene Form einnimmt.

In einer folgenden, dritten Bearbeitungseinheit, welche als Kühlelement ausgestaltet ist, kann der erwärmte Tampon-Applikator wieder abgekühlt werden. Dadurch wird der thermoplastische Kunststoff in seiner Form gehärtet.

Besonders bevorzugt sind die Bearbeitungseinheiten so ausgestaltet, dass sie eine abgeteilte Menge an Führungseinheiten zur gleichzeitigen Bearbeitung aufnehmen können. So kann z.B. eine Bearbeitungseinheit ausgestaltet sein, um gleichzeitig zwischen zwei und 24, insbesondere 36 Führungseinheiten aufzunehmen und entsprechend zu bearbeiten, also zu erhitzen, umzuformen und/oder zu kühlen. Alternativ oder ergänzend können die Bearbeitungseinheiten so ausgestaltet sein, dass sie einen kontinuierlichen Zustrom an Führungseinheiten, welche sich entlang der Führungsanordnung bewegen, durch ihren Prozessraum durchzuführen vermögen. So kann z.B. ein schrittweises Erwärmen beim Eintritt einer Führungseinheit in das Heizelement stattfinden, an dessen Austritt und Übertritt in das Umformungselement die gewünschte Temperatur erreicht wird, in der der geförderte Tampon-Applikator entsprechend umgeformt werden kann. Entsprechend kann auch die Abkühlung durch das Kühlelement kontinuierlich erfolgen.

In einer besonderen Ausführungsform ist eine Bearbeitungseinheit im Bearbeitungsraum so ausgestaltet, dass sie eine Mehrzahl an Bearbeitungsschritten auszuführen vermag. So kann eine Bearbeitungseinheit an einem einzelnen oder einer abgeteilten Menge an Tampon-Applikatoren nacheinander ein Erwärmen, ein Umformen und ein Abkühlen durchführen.

Es sind auch Mischvarianten möglich, bei denen einzelne Bearbeitungseinheiten stossweise abgeteilte Mengen bearbeiten, während andere einen kontinuierlichen Ablauf an vorbeigeführten Führungseinheiten mit entsprechenden Tampon-Applikatoren bearbeiten. So kann in einer Ausführungsform z.B. das Erwärmen durch das Heizelement kontinuierlich stattfinden, während das Umformen durch das gleichzeitige Umformen einer abgeteilten Menge durchgeführt wird. So kann z.B. das Formungselement so ausgestaltet sein, dass es zuwartet, bis eine Anzahl an erwärmten Tampon-Applikatoren mit ihren entsprechenden Führungseinheiten im Formelement angesammelt ist, bis eine Umformung mittels eines Umformungswerkzeugs durchgeführt wird, welches gleichzeitig alle im Formungselement angesammelten Tampon-Applikatoren umformt.

In einer besonderen Ausführungsform umfasst jede Führungseinheit mindestens eine Applikator-Aufnahme zur Aufnahme eines Tampon-Applikators. Bevorzugt umfasst jede Führungseinheit genau eine Applikator-Aufnahme zur Aufnahme eines Tampon-Applikators. Alternativ können die Führungseinheiten auch so ausgestaltet sein, dass sie eine Mehrzahl an Tampon-Applikatoren aufnehmen können. So wäre es auch denkbar, dass eine Führungseinheit so ausgestaltet ist, dass sie über zwei bis sechs, insbesondere drei Applikator-Aufnahmen verfügt, welche ausgelegt sind, Tampon-Applikatoren aufzunehmen.

In einer besonderen Ausführungsform sind die Applikator-Aufnahmen so ausgestaltet, dass ein Tampon-Applikator form- und/oder kraftschlüssigen Halt in der Applikator-Aufnahme findet. Dazu kann die Applikator-Aufnahme z.B. so ausgestaltet sein, dass sie der Grösse des aufzunehmenden Tampon-Applikators im Wesentlichen entspricht, oder grösser ist als der Durchmesser des Längsquerschnitts eines aufzunehmenden Tampon-Applikators, aber Elemente aufweist welche eine leichte Kraft auf den Tampon-Applikator auszuüben vermögen, wobei bevorzugt dieser sich geringfügig verformt. Zum Beispiel kann die Applikator-Aufnahme mit Haltemitteln versehen sein, z.B. einer Gummierung, einem Rippenprofil oder einer oder einer Mehrzahl an Haltebeulen, welche den Tampon-Applikator in der Applikator-Aufnahme halten. Insbesondere kann der kraftschlüssige Halt ein reibschlüssiger Halt sein.

Im Sinne der vorliegenden Erfindung kann unter einer Haltebeule eine Wölbung, Ausstülpung oder Erhebung verstanden werden, welche sich in die Applikator-Aufnahme erstreckt und das Volumen derselben begrenzt. Die Haltebeule kann auch als Dorn oder Fortsatz ausgebildet sein, der über eine elastische Spannung und/oder eine Rückstellkraft auf das Volumen der Applikator-Aufnahme dergestalt wirkt, dass ein form- und/oder kraftschlüssiger Halt auf einen darin befindlichen Tampon-Applikator ausgeübt wird.

Es wurde überraschend festgestellt, dass bereits mit einer leichten Verformung des Tampon-Applikators ein ausreichender Halt erreicht wird, um den Tampon-Applikator in der Vorrichtung zur Bestückung von Tampon-Applikatoren entlang aller vorgesehener Bearbeitungsstationen zu transportieren, ohne dass dabei die leichte Verformung die Qualität des Tampon-Applikators in Mitleidenschaft zieht, oder das Bestücken des Tampon-Applikators mit dem Tampon beeinträchtigt wird.

In einer besonderen Ausführungsform sind die Führungseinheiten mittels Rollen auf der Führungsanordnung gelagert. Zum Beispiel können die Rollen so an der Führungseinheit ausgestattet sein, dass sie in ein T-Profil einer Schiene der Führungsanordnung eingreifen.

Besonders bevorzugt weist die Führungseinheit drei Rollen auf. Mit einem System, welches drei Rollen umfasst, können die Führungseinheiten auf der Führungsanordnung fixiert werden und dabei auch Kurvenverläufen optimal folgen. In diesem Beispiel wären bei der Führungseinheit zwei Rollen auf einer Achsenseite der Führungsanordnung und eine Gegenrolle auf der Gegenseite, der hinsichtlich der Längsachse der Führungsanordnung gegenüberliegenden Seite, angeordnet.

In einer besonderen Ausführungsform umfasst die Führungsanordnung einen Aussenradius und einen Innenradius. Ist die Führungsanordnung umlaufend ausgestaltet, so beschreibt der Aussenradius einen grösseren Umfang als der Innenradius. Besonders bevorzugt sind die Führungseinheiten so auf der Führungsanordnung gelagert, dass zwei Rollen auf dem Aussenradius verlaufen und eine Rolle auf dem Innenradius verläuft. Dadurch können die Führungseinheiten auch optimal dem Kurvenverlauf der Führungsanordnung folgen. In einer weiteren besonderen Ausführungsform ist der Aussenradius auf geraden Abschnitten der Führungsanordnung parallel zum Innenradius, in den Kurvenbereichen kann der Abstand zwischen dem Innenradius und dem Aussenradius variabel ausgestaltet sein. So kann zum Beispiel ein ruhigeres Abrollen über die Kurven mit drei Rollen gewährleistet werden.

In einer alternativen Ausführungsform umfasst die Führungseinheit vier Rollen, wobei jeweils zwei Rollen als Rollenpaare auf gegenüberliegenden Radien Führungseinheit ausgebildet sind. Die Rollen eines Rollenpaares können starr zueinander ausgestaltet sein, oder auch zueinander gefedert ausgestaltet sein. Bevorzugt sind die Rollen eines Rollenpaares starr zueinander ausgestaltet, aber schwenkbar bezüglich einer Schwenkachse an der Führungseinheit montiert. Die Schwenkachse verläuft vorzugsweise parallel zur Rotationsachse der Rollen, welche mittels eines Rollenhebels miteinander verbunden sind, und erstreckt sich senkrecht durch den Rollenhebel. Mit zwei solcherart gegeneinander schwenkbar gelagerter Rollenpaare kann zum Beispiel ein ruhigeres Abrollen über die Kurven gewährleistet werden.

In einer besonderen Ausführungsform umfasst die Führungseinheit eine Haltekassette mit einer Applikator-Aufnahme. Die Haltekassette kann mit der Führungseinheit einstückig ausgestattet sein. Bevorzugt ist die Haltekassette aber bezüglich der Führungseinheit beweglich gelagert. Die Applikator-Aufnahme kann mit den verschiedenen, oben genannten Merkmalen ausgestaltet sein, welche dazu geeignet sind, einen Tampon-Applikator aufzunehmen und entsprechend form- und/oder kraftschlüssig zu halten. In der einfachsten Ausführungsform ist die Applikator-Aufnahme eine Ausnehmung, welche sich durch Haltekassette, insbesondere rechtwinklig zur Frontalebene der Haltekassette, erstreckt und einen Durchmesser aufweist, der mindestens einem Bereich der Oberfläche Tampon-Applikators physisch kontaktiert, dermassen, dass durch diesen physischen Kontakt eine form-, und/oder kraftschlüssige Verbindung zwischen Haltekassette und Tampon-Applikator besteht.

Besonders bevorzugt ist die Haltekassette einstückig ausgebildet.

In einer besonderen Ausführungsform ist die Haltekassette aus einem Material, welches einen geringen Abrieb bei Reibungsbelastungen aufweist. Insbesondere ist die Haltekassette aus einem Kunststoff, z.B. aus einem Nylon-Kunststoff.

In einer besonderen Ausführungsform umfasst die Führungseinheit ein Führungselement, welches Eingreifmittel aufweist, die mit der Führungsanordnung in Wirkverbindung treten können, sodass eine verschiebbare Lagerung der Führungseinheit auf der Führungsanordnung besteht.

In einer weiteren besonderen Ausführungsform ist die Haltekassette hinsichtlich des Führungselements bewegbar gelagert. In dieser Ausführungsform kann die Führungseinheit im Wesentlichen in zwei funktionale Elemente unterteilt werden. Die Haltekassette dient der Aufnahme, der Lagerung und der Weitergabe der Tampon-Applikatoren in der Vorrichtung, während das Führungselement der Lagerung und der Verschiebbarkeit der Führungseinheit auf der Führungsanordnung dient.

In einer besonderen Ausführungsform sind die Eingreifmittel Rollen, wie oben geschildert. Alternativ kann das Eingreifmittel allerdings auch mit einem Gleitlager ausgestattet sein, welches mit einem entsprechenden Gegenstück der Führungsanordnung in Wirkverbindung treten kann, sodass dieses entlang der Längsachse der Führungsanordnung verschiebbar ist.

In einer besonderen Ausführungsform ist die Haltekassette hinsichtlich des Führungselements so bewegbar gelagert, dass die Haltekassette hinsichtlich des Führungselements gefedert ist. Zum Beispiel kann ein Federelement zwischen der Haltekassette und dem Führungselement angeordnet sein, sodass eine Rückstellkraft auf die Haltekassette ausgeübt wird.

In einer weiteren besonderen Ausführungsform ist die Haltekassette so bewegbar gelagert, dass sie im Wesentlichen rechtwinklig zur Längsachse der Führungsanordnung bei montierter Führungseinheit bewegbar gelagert ist. Die Bewegung der Haltekassette geschieht in diesem Beispiel somit in radialer Richtung bezüglich eines Umlaufradius der Führungsanordnung. Die Rückstellkraft des Federelements kann so ausgestaltet sein, dass einerseits eine Bewegung der Haltekassette in radialer Richtung, d.h. ein Wegziehen der Haltekassette vom Führungselement durch die Rückstellkraft kompensiert wird, oder andererseits eine Verschiebung der Haltekassette in Richtung des Führungselements zu einer Stauchung der Feder führen würde und die Rückstellkraft diese Bewegung kompensiert. Bevorzugt ist ein Rückstellbereich zwischen der Haltekassette und dem Führungselement ausgelassen, sodass die Bewegung der Haltekassette ermöglicht wird. Besonders bevorzugt ist die Rückstellfeder in diesem Rückstellbereich oder benachbart angebracht. Anstelle einer Feder kann auch ein Gummiband mit einem entsprechenden elastischen Kunststoff für die nötige Rückstellkraft sorgen

In einer besonderen Ausführungsform umfasst die Führungsanordnung entlang ihrer Umlaufbahn mindestens ein Antriebselement, welches dazu ausgelegt ist, mindestens eine Führungseinheit in eine Förderrichtung zu befördern. Dazu kann das Antriebselement z.B. einen Mitnehmer aufweisen, der mit der Führungseinheit in Wirkverbindung treten kann, damit das Antriebselement die Führungseinheit auf mindestens einem Teil der Führungsanordnung zu bewegen vermag. Alternativ sind die Führungseinheiten selbst angetrieben. Dies kann z.B. über einen Antrieb an einer der besagten Rollen ermöglicht werden.

In einer besonderen Ausführungsform sind eine Mehrzahl von Antriebselementen entlang der Umlaufbahn der Führungsanordnung positioniert. Insbesondere können sich angetriebene Bereiche von nicht angetriebenen Bereichen unterscheiden, so dass die Führungseinheiten zeitweise aktiv in Förderrichtung bewegt werden und zu anderen Zeitpunkten passiv sich in Förderrichtung bewegen, z.B. indem sie von anderen Führungseinheiten angestossen werden, oder indem sie Schwerkraft als Antrieb nutzen bei einer entsprechenden geometrischen Anordnung der Führungsanordnung. So kann z.B. die Führungsanordnung vertikal angeordnet sein, so dass die umlaufende Führungsanordnung ein Gefälle aufweist, auf welchem die Führungseinheiten durch die Schwerkraft nach unten gezogen werden und eine Beschleunigung erfahren.

In einer besonderen Ausführungsform weist ein Antriebselement eine Mehrzahl an Mitnehmern auf, um gleichzeitig eine Mehrzahl von Führungselementen in Förderrichtung zu befördern. So kann z.B. mittels eines Mitnehmers ein Antriebselement eine abgeteilte Menge an Führungselementen gleichzeitig antreiben.

Die Antriebselemente können auch so ausgestaltet sein, dass sie selbst umlaufend wirksam sind, so dass eine kontinuierliche Förderung in eine Förderrichtung ermöglicht wird. Auf dem umlaufenden Radius können die Antriebselemente eine Reihe von Mitnehmern aufweisen, die bei Kontakt eine Führungseinheit erfassen und sich in Förderrichtung bewegen, bis sich diese Führungseinheit ausserhalb des Einflussbereiches des entsprechenden Antriebselements bewegt, worauf sie entweder von einem nachfolgenden Antriebselement weitergefördert würde, oder einer bestimmten Bearbeitung zugeführt würde. Dabei kann das Antriebselement bereits weitere Führungseinheiten durch weitere Mitnehmer aufgenommen haben.

In einer besonderen Ausführungsform umfasst die erfindungsgemässe Vorrichtung eine Steuerung zur Synchronisation der Antriebselemente mit den entsprechenden Bearbeitungseinheiten. Durch die Steuerung der Antriebselemente und der Bearbeitungseinheiten kann z.B. eine Pufferung erreicht werden, wenn bei der Bearbeitung ein Rückstau entstehen könnte.

In einer besonders bevorzugten Ausführungsform ist die Steuerung dynamisch und reagiert auf die Geschwindigkeit der einzelnen Elemente, also zum Beispiel der Bearbeitungseinheiten und der Antriebselemente. Dabei kann z.B. die Geschwindigkeit einzelner Elemente an Verzögerungen oder Beschleunigungen in den anderen Elementen dynamisch angepasst werden.

In einer besonderen Ausführungsform ist die Bestückungseinheit trommelförmig aufgebaut und weist eine Mehrzahl von radial angeordneter Bestückungsmitnehmer auf, die je zur Mitnahme eines Mitnahmeansatzes der Führungseinheiten ausgestaltet sind. Der Bestückungsmitnehmer kann z.B. eine Rippe oder eine Ausnehmung mit einem Profil sein, welche mit einem entsprechenden Gegenstück an der Führungseinheit in Wirkverbindung treten können, wenn die Führungseinheit den Bearbeitungsbereich der Bestückungseinheit betritt. Der Mitnahmeansatz der Führungseinheit kann ebenfalls so ausgestattet sein, wie es der entsprechende Bestückungsmitnehmer erfordert. In der einfachsten Ausführungsform ist der Mitnahmeansatz ein Stift, welcher von einem halbkreisförmigen Bestückungsmitnehmer der Bestückungseinheit mitgenommen wird.

Somit ist es möglich, die Zufuhr der Führungseinheiten präzise zu steuern, was der späteren Bestückung der Tampon-Applikatoren in den Applikator-Aufnahmen zugutekommt. So wird insgesamt ein flexibler, hochgradig steuerbarer und schonender Prozess zur Bestückung von Tampon-Applikatoren mit Tampons bereitgestellt.

Ein weiterer Vorteil kann realisiert werden, wenn die Führungseinheiten im Wesentlichen zweistückig ausgebildet sind, d.h. das Führungselement und die Haltekassette zwei separate Bauteile sind. Die Haltekassette ist durch die Bestückung mit Tampon-Applikatoren und durch die Eingreifwirkung der Mitnahmeansätze der Bestückungseinheit stärkerem Verschleiss ausgesetzt. Die Haltekassette kann mit einem geringeren Materialverlust leicht ersetzt werden. Ausserdem kann die Haltekassette an die entsprechenden Dimensionen eines Tampon-Applikators angepasst werden. So sind z.B. Tampon-Applikatoren für Tampons, welche in den Zwischenmenstruationstagen genutzt werden, im Durchmesser kleiner als die Tampon-Applikatoren, die Tampons zur Verwendung während der Regelblutung aufweisen. Beispiele für solche, kleiner dimensionierte Tampons sind in der EP 2'869'802 B1 beschrieben.

In einer besonderen Ausführungsform folgt die Führungsanordnung im Bereich der Bestückungsstation einem Radius, welcher parallel zum Rotationsradius eines Bestückungsbereichs der Bestückungsstation verläuft. Zum Beispiel kann die Führungsanordnung in diesem Bereich von der grundsätzlich ovalen Grundform abweichen und dem Radius der Bestückungseinheit folgen. Zum Beispiel kann die Bestückungseinheit im Wesentlichen kreisrund ausgestaltet sein mit den entsprechenden radial angeordneten Bearbeitungsbereichen. Die Führungsanordnung kann von einem linearen Bereich eine Kurve beschreiben, welche dann einem Radius folgt, sodass die Führungsanordnung dem Umfang der Bestückungseinheit in diesem Bereich folgt. Eine neuerliche Kurve kann die Führungsanordnung wieder in einen linearen Bereich überführen.

In einer besonderen Ausführungsform umfasst die Bestückungsstation eine Mehrzahl von radial angeordneten Anschlagsbereichen zur koaxialen Ausrichtung der Applikator-Aufnahme einer Führungseinheit mit einer Tampon-Aufnahme der Bestückungsstation, sodass ein Tampon aus der Tampon-Aufnahme entlang einer Übertragungsachse in die Applikator-Aufnahme übertragen werden kann. Die koaxiale Ausrichtung der Applikator-Aufnahme im Sinne der vorliegenden Erfindung gilt hinsichtlich der Längsachse der Applikator-Aufnahme. So kann z.B. eine Haltekassette mit einer Ausnehmung als Applikator-Aufnahme ausgestattet sein, welche sich durch die ganze Haltekassette hindurch erstreckt. Diese Ausnehmung weist eine Längsachse auf, die im Wesentlichen der Längsachse eines aufzunehmenden Tampon-Applikators entspricht. Diese Längsachse wird in eine koaxiale Ausrichtung zur Tampon-Aufnahme einer Bestückungsstation gebracht, sodass ein Tampon in einen Tampon-Applikator überführt werden kann. Dies kann z.B. mittels eines Stössels aktiv erfolgen.

Im Betrieb kann z.B. in der Ausführungsform mit der gefederten Haltekassette eine Verschiebung der Haltekassette in radialer Richtung etwaige Ungenauigkeiten kompensieren, resp. eine entsprechende koaxiale Anordnung erleichtern.

So kann z.B. in einem entspannten Zustand, wenn die Haltekassette auf der Führungsanordnung transportiert wird, die Rückstellkraft ungehindert in radialer Richtung wirken. Wird nun die Führungseinheit mit der entsprechenden Haltekassette in den Wirkbereich eines Anschlagsbereichs gebracht, so wird eine Kraft gegen die radiale Kraft der Rückstellkraft, welche auf die Haltekassette wirkt, auf diese ausgeübt. Die Rückstellkraft kann zu diesem Zeitpunkt sicherstellen, dass die Haltekassette und die Applikator-Aufnahme in Bezug auf die Bestückungseinheit korrekt ausgerichtet sind.

Mit der erfindungsgemässen Vorrichtung ist es möglich, eine Bestückung von Tampon-Applikatoren in einem kontinuierlichen Herstellungsprozess von in Applikatoren verpackten Tampons zu ermöglichen. Die erfindungsgemässe Vorrichtung weist besondere Vorteile in ihrer Skalierbarkeit, sowie der Modularität einzelner Bestandteile auf. So ist es z.B. möglich, die Bestückung an die entsprechende Zufuhr von Tampon-Applikatoren oder an die Bearbeitungsgeschwindigkeit der Bestückungseinheit anzupassen. Die hohe Modularität erlaubt es auch, besonders preisgünstig Verschleissteile einzeln auszuwechseln, ohne dass dabei wesentliche Bestandteile der Vorrichtung ersetzt werden müssen. Durch die Führungseinheiten mit der Haltekassette ist ein besonders präzises Bestücken der Tampon-Applikatoren möglich, was auch hohen Qualitätsanforderungen entspricht. So wird ein Tampon mit der erfindungsgemässen Vorrichtung wenig bis gar nicht ausfransen während der Bestückung. Gleichzeitig ist ein schonender Transport der Tampon-Applikatoren gewährleistet, was zu einem optisch einwandfreien Produkt führt, welches bei den Anwendern eine hohe Akzeptanz erfährt.

Ein weiterer Aspekt der vorliegenden Erfindung betrifft eine Führungseinheit zum Transport von Tampon-Applikatoren auf einer umlaufenden Führungsanordnung, insbesondere einer Führungsanordnung gemäss der oben beschriebenen Vorrichtung. Die Führungseinheit umfasst eine Applikator-Aufnahme zur Aufnahme eines Tampon-Applikators. Die Applikator-Aufnahme weist Mittel zum form-, und/oder kraftschlüssigen Halt des Tampon-Applikators in der Applikator-Aufnahme auf. Diese Applikator-Aufnahme-Mittel können beispielsweise durch die Form der Applikator-Aufnahme oder durch entsprechende Strukturen an der Applikator-Aufnahme gewährleistet werden. Beispiele für die Ausgestaltung der Applikator-Aufnahme wurden bereits oben geschildert. Zusätzlich seien als Varianten noch mögliche Gummierungen, Aufrauungen, Federelemente und/oder Haltebeulen erwähnt. Auch eine Kombination verschiedener solcher Mittel zum form- und/oder kraftschlüssigen Halt der Tampon-Applikatoren sind denkbar.

Die erfindungsgemässe Führungseinheit weist zudem ein Führungselement mit Eingreifmitteln zur gelagerten Verschiebung der Führungseinheit auf der umlaufenden Führungsanordnung auf. Die Eingreifmittel sind bevorzugt so ausgestaltet, dass sie auch einem Kurvenverlauf der umlaufenden Führungsanordnung folgen können. Die Eingreifmittel können starr sein, oder sie können bezüglich der Längsachse der Führungsanordnung bewegbar gelagert sein, z.B. indem sie Federelemente aufweisen.

In der einfachsten Ausführungsform sind die Eingreifmittel Rollen oder ein Gleitlager. Auch eine Kombination aus Rollen und Gleitelementen ist denkbar. So können z.B. zwei Laufrollen auf einer Seite in eine Schiene der Führungsanordnung eingreifen, während auf der anderen Seite ein Gleitlager für den notwendigen Gegendruck sorgt, damit die Rollen innerhalb der Schiene gelagert sind.

Die erfindungsgemässe Führungseinheit weist zudem mindestens einen Mitnahmeansatz auf, der mit einer Bestückungseinheit und/oder mit einem Antrieb in Wirkverbindung treten kann.

In einer besonderen Ausführungsform ist die Applikator-Aufnahme hinsichtlich des Führungselements verschiebbar gelagert. Bevorzugt ist sie in radialer Richtung bezüglich der umlaufenden Führungsanordnung verschiebbar gelagert, wie das z.B. oben ausgeführt ist.

In einer besonderen Ausführungsform umfasst die Führungseinheit eine Mehrzahl an Mitnehmern. So kann die Führungseinheit neben des Mitnahmeansatzes, welches zur Wechselwirkung mit der Bestückungseinheit vorgesehen ist, mindestens einen Stift aufweisen, der zur Wechselwirkung mit einem Antriebselement ausgestaltet ist.

In einer besonderen Ausführungsform umfasst die Führungseinheit eine Haltekassette, welche zur Aufnahme eines Tampon-Applikators ausgelegt ist.

In einer besonders bevorzugten Ausführungsform besteht die Applikator-Aufnahme aus einer Ausnehmung, welche in der Haltekassette ausgebildet ist, und deren Querschnitt rechtwinklig zur Längsausdehnung der Ausnehmung eine Mehrzahl an Aus- und/oder Einbuchtungen umfasst. Besonders bevorzugt umfasst die Haltekassette eine Applikator-Aufnahme, die mindestens eine, vorzugsweise eine bis vier, besonders bevorzugt drei Haltebeulen aufweist, welche sich in die Ausnehmung der Applikator-Aufnahme erstrecken, sodass die Haltebeule(n) bei einem eingeführten Tampon-Applikator zu einer form- und/oder kraftschlüssigen Haltung des Tampon-Applikators in der Applikator-Aufnahme führen.

Ein weiterer Aspekt der vorliegenden Erfindung betrifft ein Verfahren zur Bestückung von Tampon-Applikatoren mit Tampons. Bevorzugt wird das erfindungsgemässe Verfahren mit einer eingangs geschilderten Vorrichtung und mit oben beschriebenen Führungseinheiten durchgeführt. Das Verfahren umfasst im Wesentlichen einen ersten Schritt des Bereitstellens einer Führungseinheit zum Transport von Tampon-Applikatoren auf einer umlaufenden Führungsanordnung. In einer besonderen Ausführungsform wird bei diesem Schritt durch jede Führungseinheit ein einzelner Tampon-Applikator auf der umlaufenden Führungsanordnung befördert. Das erfindungsgemässe Verfahren umfasst weiter ein Bestücken einer Applikator-Aufnahme der Führungseinheit mit einem Tampon-Applikator an einer Eingangsstelle. Dazu können z.B. durch ein Tampon-Applikator-Zuführmittel Tampon-Applikatoren mit einem Stössel in eine Ausnehmung einer Führungseinheit geschoben werden, sodass diese mit einer form- und/oder kraftschlüssigen Verbindung in diesen während des Transportes gehalten werden. Die Eingangsstelle kann z.B. so ausgestaltet sein, dass die Tampon-Applikatoren an bewegte Führungseinheiten übergeben werden. Alternativ können die Führungs-Einheiten an einer Eingangsstelle kurzfristig parkiert werden, während sie mit den Tampon-Applikatoren bestückt werden.

In einer besonderen Ausführungsform ist ein Bestückungsmittel zur Bestückung der Führungseinheiten so ausgestaltet, dass sie mindestens über einen Teilbereich der Führungsanordnung die Bewegung der Führungseinheiten mitmacht, sodass eine Übergabe der Tampon-Applikatoren an die Führungseinheiten in der Bewegung vollführt werden kann.

Das erfindungsgemässe Verfahren umfasst weiter ein Verschieben der Führungseinheit auf der Führungsanordnung von der Eingangsstelle zu einer Bestückungseinheit. Dieses Verschieben kann, wie bereits oben geschildert, passiv oder aktiv durch die Führungseinheit selbst erfolgen. In der einfachsten Ausführungsform wird dieses Verschieben durch ein Antriebselement, ebenfalls wie beschrieben, ermöglicht.

Das erfindungsgemässe Verfahren umfasst weiter ein Bestücken des Tampon-Applikators mit einem Tampon. Dieses Bestücken kann mit einer beschriebenen Bestückungseinheit erfolgen.

Das erfindungsgemässe Verfahren umfasst weiter ein Verschieben der Führungseinheit auf der Führungsanordnung von der Bestückungseinheit zu einer Ausgangsstelle, wo der bestückte Tampon-Applikator ausgeschieden werden kann. Dieses Ausscheiden kann analog wie die Eingangsstelle dynamisch erfolgen, d.h. mit einer Ausscheidungseinheit, welche der Bewegung der Führungseinheiten im Wesentlichen folgt und dabei das Ausscheiden durchführt, oder in einem kurzfristig parkierten Zustand, indem die Führungseinheit angehalten wird und ein Ausstoss des bestückten Tampon-Applikators erfolgt.

In einer besonderen Ausführungsform des erfindungsgemässen Verfahrens wird beim Bestücken des Tampon-Applikators mit einem Tampon die Applikator-Aufnahme der Führungseinheit in eine bezüglich ihrer Längsachse koaxiale Ausrichtung zu einer Tampon-Aufnahme der Bestückungseinheit geführt.

In einer weiteren Ausführungsform greift die Bestückungseinheit mittels mindestens eines Bestückungsmitnehmers einen Mitnehmeransatz der Führungseinheit ein und führt diese so in die Übergabeposition.

In einer erfindungsgemässen Ausgestaltung können alle oben genannten Merkmale in einer beliebigen Kombination verwirklicht werden, sofern sie sich nicht gegenseitig ausschliessen.

Im folgenden Abschnitt wird die vorliegende Erfindung nun anhand konkreter Figuren und Ausführungsbeispiele näher erläutert, ohne auf diese beschränkt zu sein.

Für einen Fachmann ergeben sich aus diesen detaillierten Beschreibungen weitere vorteilhafte Merkmale, welche in einer Ausgestaltung der vorliegenden Erfindung verwirklicht sein können.

### Figurenbeschrieb

Anhand der nachfolgenden Figuren werden Ausführungsbeispiele der Erfindung beschrieben.

Es zeigt:
- Fig. 1: schematisch eine Ausführung der erfindungsgemässen Vorrichtung,
- Fig. 2: schematisch im Längsquerschnitt einen bestückten Tampon-Applikator;
- Fig. 3a: schematisch eine bestückte Führungseinheit;
- Fig. 3b: schematisch eine Führungseinheit in Frontalansicht;
- Fig. 4: schematisch einen Mitnehmer für eine abgeteilte Menge an Führungseinheiten;
- Fig. 5a: schematisch eine Bestückungseinheit und ihr zugehöriger Bearbeitungsraum einer Führungsanordnung;
- Fig. 5b: schematisch eine Wirkverbindung zwischen einer Führungseinheit und der Bestückungseinheit;
- Fig. 6a: schematisch eine Ausgestaltung einer Haltekassette;
- Fig. 6b: eine weitere schematische Ausgestaltung einer Haltekassette.

### Ausführung der Erfindung

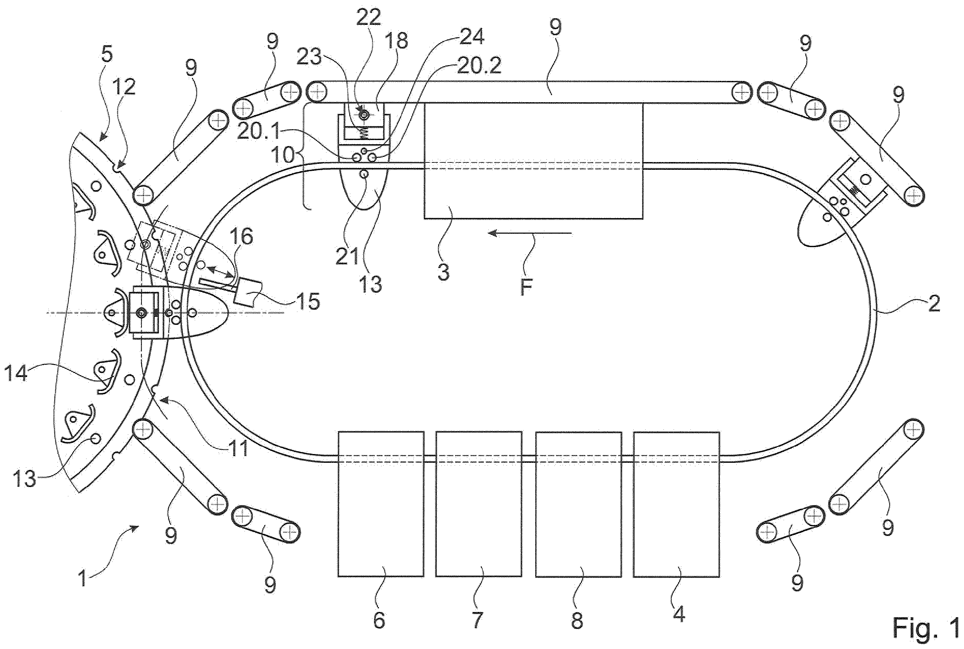

Fig. 1 zeigt schematisch eine Vorrichtung 1 zur Bestückung von Tampon-Applikatoren mit Tampons in einer möglichen Verwirklichung der vorliegenden Erfindung. Die Vorrichtung zur Bestückung von Tampon-Applikatoren organisiert sich um eine umlaufende Führungsanordnung 2. In diesem Beispiel ist die Führungsanordnung 2 als langgestrecktes Rechteck angeordnet, dessen beide Schmalseiten durch Halbkreise ersetzt sind. Die Führungsanordnung 2 umfasst eine Schiene (schematisch gezeigt), auf der sich eine Reihe von Führungseinheiten 10 gelagert bewegen können. Im vorliegenden Beispiel sind insgesamt vier Führungseinheiten 10 zur beispielhaften Illustration gezeigt.

In einer Verwirklichung der vorliegenden Erfindung können zwischen vier und 4.000, insbesondere zwischen acht und 2.000, weiter insbesondere zwischen 24 und 400 Führungseinheiten 10 auf einer Führungsanordnung 2, wie sie in der Fig. 1 gezeigt ist, eingesetzt werden. Die Anzahl der Führungseinheiten 10 kann an die Bearbeitungsgeschwindigkeit oder die Zufuhr an Tampon-Applikatoren angepasst werden. Die Führungseinheiten 10 dienen dem Transport einzelner Tampon-Applikatoren entlang des Umfangs der Führungsanordnung 2. Auf diesem Umfang der Führungsanordnung 2 sind verschiedene Bearbeitungsstationen angeordnet, welche einen Arbeitsschritt an den Tampon-Applikatoren vornehmen können.

Die Tampon-Applikatoren werden an einer Eingangsstelle 3 in eine Applikator-Aufnahme 22 einer Führungseinheit 10 eingeführt. Dazu kann z.B. eine abgeteilte Menge an bereitgestellten Tampon-Applikatoren mittels eines Stössels in eine entsprechende Anzahl an Führungseinheiten 10 überführt werden (nicht gezeigt). Die in diesem Beispiel erläuterte Vorrichtung 1 weist eine Förderrichtung F auf, in die die Führungseinheiten 10 mittels Antriebselementen 9 oder nicht gezeigter anderer Antriebselemente in den einzelnen Bearbeitungsstationen bewegt werden. Dazu verfügen die Führungseinheiten 10 über an der Schiene der Führungsanordnung 2 gelagerte Rollen 20.1, 20.2, 21. Die beispielhaft gezeigte Führungseinheit 10 nahe der Eingangsstelle 3 verfügt über eine erste Rolle 20.1 und eine zweite Rolle 20.2, welche am äusseren Umfang der Führungsanordnung 2 gelagert sind, und eine Gegenrolle 21, welche im inneren Umfang der Führungsanordnung 2 gelagert ist. Dazu kann die Führungsanordnung 2 eine als T-Profil ausgestaltete Schiene aufweisen, in die die Rollen 20.1, 20.2, 21 eingreifen können. Die Führungsanordnung kann zudem über ihrem Umfang variable Radien für den Aussenradius und den Innenradius beschreiben, so können zum Beispiel parallele Strecken auf den Geraden in variablen Radien an den Kurven übergehen, um ein besseres Abrollen der Führungseinheiten über den Kurven zu ermöglichen.

Die Rollen sind rotierbar gelagert an einem Führungselement 19 angeordnet. Im vorliegenden Beispiel ist dieses Führungselement 19 bezüglich der Führungsanordnung 2 starr. Alternativ kann das Führungselement 19 über gefederte Rollen 20.1, 20.2, 21 hinsichtlich der Führungsanordnung 2 bewegbar gelagert sein. Gefederte Rollen würden über eine Rückstellkraft in im Wesentlichen normaler Richtung hinsichtlich der Führungsanordnung 2 bewegbar sein. Im vorliegenden Kontext sei als normale Richtung eine Richtung senkrecht zur Tangente der Führungsanordnung zu verstehen. Im vorliegenden starren Beispiel ist das Führungselement 19 mit einer gefederten Haltekassette 18 verbunden, welche die Applikator-Aufnahme 22 aufweist. Eine Rückstellfeder 23 zwischen der Haltekassette 18 und dem Führungselement 19 sorgt dafür, dass die Haltekassette 18 senkrecht zu einer Tangente der Führungsanordnung 2 bewegbar gelagert ist. Im vorliegenden Beispiel sind insgesamt neun Antriebselemente 9 gezeigt. Die Anzahl der Antriebselemente kann allerdings an die Geometrie der Führungsanordnung 2 bei Bedarf angepasst werden. Im vorliegenden Beispiel sind alle Antriebselemente zudem über einen Kopfantrieb einer Antriebsrolle angetrieben. Die Antriebselemente 9 können auch gebündelt angetrieben werden, indem sie z.B. mit Antriebsbändern über eine Welle verbunden sind. Somit würde ein einzelner Antriebsmotor reichen, um eine für die Ausführung der vorliegenden Erfindung erforderlichen Anzahl an Antriebselementen 9 anzutreiben. Die Antriebselemente 9 greifen die Führungseinheiten 10 über Mitnehmer (nicht gezeigt in der Fig. 1) und bewegen sie in Förderrichtung F.

In der in Förderrichtung F nachgelagerten ersten Schmalseite beginnt der Bearbeitungsbereich der Bestückungseinheit 5. Die Bestückungseinheit 5 ist in der Fig. 1 nur ausschnittsweise gezeigt und insgesamt rotationssymmetrisch und trommelförmig ausgebildet, sodass sie radial angeordnete Tampon-Aufnahmen 13 aufweist. Gelangt eine Führungseinheit 10 in den Bearbeitungsraum der Bestückungseinheit 5, sorgt ein Bestückungsmitnehmer 12 der Bestückungseinheit 5 dafür, dass die Führungseinheit 10 mitgenommen wird und über einen Ausrichtradius 11 geführt wird, bei dem die Applikator-Aufnahme 22 der Führungseinheit 10 mit der Tampon-Aufnahme 13 im Wesentlichen konzentrisch ausgerichtet ist und die Bestückung stattfinden kann. Bei der konzentrischen Anordnung sorgt ein Anschlagbereich 14 der Bestückungseinheit 5 für eine Kompression der Rückstellfeder 23, welche die Haltekassette 18 hin zum Führungselement 19 verschiebt. Durch die Rückstellkraft der Rückstellfeder 23 wird die Ausrichtung vervollständigt. Die Bestückungsmitnehmer 12 treten bei der Mitnahme der Führungseinheiten 10 mit einem am Führungselement 24 als Stift ausgebildeten Mitnahmeansatz in Wirkverbindung.

Um die Mitnahme der Führungseinheiten 10 an der Bestückungseinheit 5 optimal zu steuern, verfügt die Vorrichtung 1 zusätzlich über eine Vereinzelungseinheit 15 mit einem verschiebbaren Stopper 16, welche die Führungseinheiten 10 an die Frequenz der Bestückungseinheit 5 anpassen kann.

Der Bestückungseinheit 5 sind eine Reihe von Bearbeitungseinheiten 6, 7, 8 nachgelagert. Über Antriebselemente 9 gelangt eine Führungseinheit 10 in die Bearbeitungseinheiten 6, 7, 8. Dies kann kontinuierlich erfolgen oder über abgeteilte Mengen an Führungseinheiten 10, welche gesammelt und über einen Mitnehmer in die einzelnen Bearbeitungseinheiten 6, 7, 8 überführt werden. Im vorliegenden Beispiel ist die erste Bearbeitungseinheit ein Heizelement, bei dem die Tampon-Applikatoren auf eine Temperatur von ca. 100 Grad C erwärmt werden und somit eine Formbarkeit erhalten, welche es der nachfolgenden zweiten Bearbeitungseinheit, dem Formungselement, ermöglichen, das distale Ende des Tampon-Applikators umzuformen, sodass es sich wie eine Knospe um den Tampon schliesst. Die folgende Bearbeitungseinheit ist ein Kühlelement 8, welches den Tampon-Applikator wieder auf eine Bearbeitungstemperatur von ca. 20 °C herunterkühlt und dabei die Umformung festigt.

An einer Ausgangsstelle 4 werden die fertig bestückten Tampon-Applikatoren aus der Vorrichtung 1 ausgeschieden und einer allfälligen weiteren Verarbeitung zur Konfektionierung oder Verpackung zugeführt.

In der beispielhaft gezeigten Ausführungsform kann ein Pufferbereich 29 der Bestückungseinheit 5 nachgelagert sein. Dieser Pufferbereich 29 kann passiv ausgelegt sein, das heisst ein Bereich in dem die Führungseinheiten sich passiv ansammeln, oder er kann aktiv gepuffert sein, indem zum Beispiel ein oder mehrere Stopper eine gewisse Anzahl an Führungseinheiten anhäufen. Ist eine definierte Anzahl an Führungseinheiten im Pufferbereich 29 gesammelt worden, so können diese stossweise oder kontinuierlich, zum Beispiel über einen Mitnehmer, weitergefördert werden. Dies kann als abgeteilte Menge geschehen, oder über einzelne Mitnehmer für die Fördereinheiten. Im vorliegenden Beispiel ist ein Pufferbereich 29 vorgesehen, der der Bestückungseinheit 5 nachgelagert ist, aber der ersten Bearbeitungseinheit 6 der Reihe von Bearbeitungseinheiten 6, 7, 8 vorgelagert ist, so dass dort eine Anzahl an Führungseinheiten gesammelt und stossweise an die Bearbeitungseinheit übergeben werden kann.

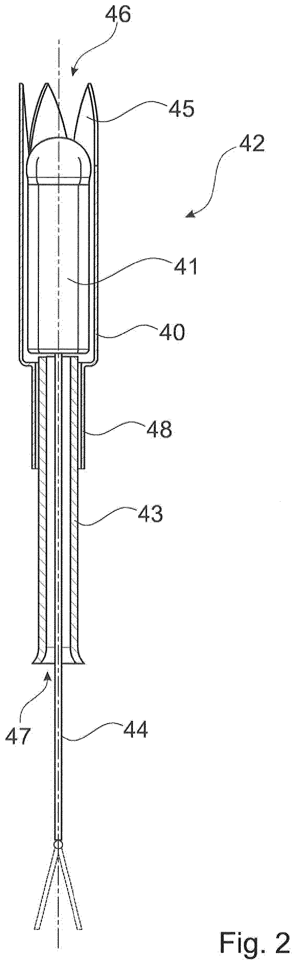

In der Fig. 2 wird ein Beispiel eines bestückten Tampon-Applikators gezeigt. Dabei ist der bestückte Tampon-Applikator 42 im Längsquerschnitt mit eingesetztem Tampon 41 dargestellt. Der bestückte Tampon-Applikator 42 verfügt über ein distales Ende 46, welches den Applikator-Kopf bildet, und ein proximales Ende 47, welches eine Öffnung für einen Rückholfaden 44 aufweist. Dieser Rückholfaden 44 erstreckt sich bis zum eingesetzten Tampon 41, welcher im Tampon-Körper 40 im Wesentlichen passgenau angeordnet ist. Hin zum proximalen Ende vom Tampon-Körper 40 verjüngt sich der Tampon-Applikator in einen integral mit dem Tampon-Körper 40 ausgebildeten Griffbereich 48, der wahlweise auch eine Gummierung oder Aufrauung oder anderweitige Beschaffenheit der Oberfläche aufweisen kann, welche eine Handhabung erleichtern. Am distalen Ende 46 befindet sich die Kopföffnung 45 mit einer Mehrzahl an blütenblätterförmigen Vorsprüngen. Im vorliegenden Beispiel ist der bestückte Tampon-Applikator 42 noch in seiner offenen Form, d.h. vor der Umformung durch ein Formungselement 7 dargestellt. Bei der Umformung wird das proximale Ende und die Kopföffnung 45 verschlossen, indem die Vorsprünge nach innen zur zentralen Längsachse des Tampon-Applikators umgebogen werden.

Der hier gezeigte Tampon-Applikator soll nur als Beispiel für einen für die erfindungsgemässe Vorrichtung geeigneten Tampon-Applikator gesehen werden. Andere Geometrien bezüglich der Kopföffnungen, wie ein einfacher Querschnitt, eine offene Kopföffnung (benötigt keine Umformungselemente) oder ein spiralförmig eingeschnittenes Kopfteil wären ebenso denkbar. Auch können mehrteilige Tampon-Applikatoren vorgesehen sein, bei denen das Kopfende nicht angeformt werden muss, sondern als separate Kappe in einem weiteren Bearbeitungsschritt auf den Tampon-Körper aufgesetzt und z.B. verrastet wird. Auch alternative Ausführungsformen, welche nicht thermoplastische Materialien, wie z.B. einen Karton oder einen Duroplasten, umfassen, können anderweitige Umformungen oder Abschlussschritte umfassen, um den Tampon-Kopf zu schliessen.

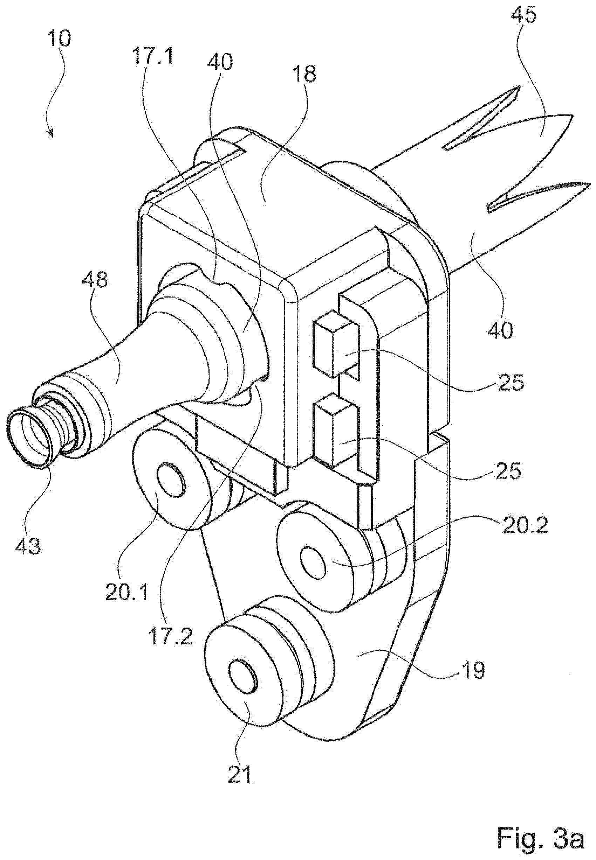

Die Fig. 3a zeigt schematisch den Aufbau einer Führungseinheit 10. Im vorliegenden Beispiel ist die Führungseinheit 10 mit einem aufgenommenen Tampon-Applikator gezeigt. Der Tampon-Applikator wird am Tampon-Körper 40 von drei an einer Haltekassette 18 ausgebildeten Haltebeulen 17.1, 17.2 mittels einer form- und/oder kraftschlüssigen Verbindung gehalten. Die Haltebeulen 17.1, 17.2 erstrecken sich gerade sowie in eine Applikator-Aufnahme der Haltekassette 18, dass der eingesetzte Tampon-Applikator an seinem Tampon-Körper 40 gerade soweit gestaucht wird, dass er sicher gehalten wird, ohne Materialschäden zu erleiden. Der Tampon-Applikator ist so in die Applikator-Aufnahme eingeführt, dass sein proximales Ende mit dem Stössel 43 zu einer Frontseite weist, und das Kopfende 45 auf eine Rückseite weist.

Der Stössel 43 ist nicht einstückig mit dem Tampon-Körper 40 ausgebildet und ist in Längsrichtung des Tampon-Applikators verschiebbar. Beim Einführen des Tampons in den Tampon-Applikator und Bestücken desselben, wird der Stössel 43 aus dem Tampon-Körper hinausgedrückt und nimmt die in der Fig. 2 gezeigte Konfiguration ein.

Die Haltekassette 18 ist als separates Bauteil der Führungseinheit 10 ausgebildet und ist über Rastnasen 25 mit einem Führungselement 19 verbunden. Am Führungselement 19 sind insgesamt drei Rollen 20.1, 20.2 und 21 ausgebildet. Im Betrieb greifen die Rollen 20.1, 20.2, 21 in ein T-Profil einer Schiene einer Führungsanordnung ein, sodass die erste Rolle 20.1 und die zweite Rolle 20.2 auf einer Seite abrollen, insbesondere auf dem Aussenradius der Führungsanordnung und die Gegenrolle 21 den Innenradius der Führungsanordnung abrollt.

Die Haltekassette 18 kann hinsichtlich des Führungselements bewegbar gelagert sein. Dazu sind die Rastnasen 25 so ausgebildet, dass unterhalb der Rastnasen 25 ein Bewegungsspielraum besteht, durch welche die Haltekassette 18 hinsichtlich des Führungselements 19 verschoben werden kann. In dieser Darstellung ist keine Feder oder anderweitiges Rückstellelement dargestellt, kann aber, wie eingangs geschildert, ebenso verwirklicht sein. Die Haltekassette 18 kann in der vorliegenden Vorrichtung einfach ausgewechselt werden. Da die Haltekassette 18 einen erhöhten Verschleiss durch die Kontaktierung der Tampon-Applikatoren erfahren kann, ist mit der vorliegenden Führungseinheit 10 zudem eine ökonomische und ökologische Verbesserung erreicht.

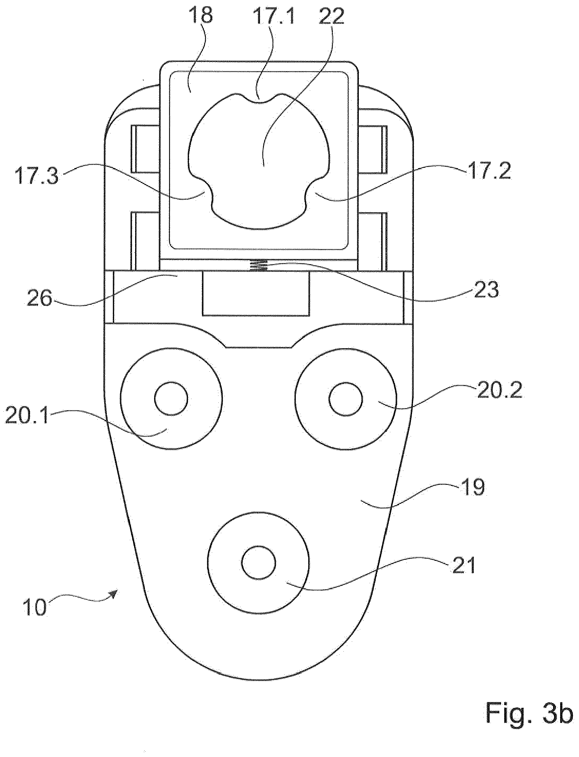

In der Fig. 3b ist die Führungseinheit 10 in Aufsicht dargestellt. Die Führungseinheit 10 unterteilt sich grob in zwei funktionale Elemente, die Haltekassette 18 und das Führungselement 19. Im vorliegenden Beispiel ist das Führungselement 19 mit drei Rollen 20.1, 20.2 und 21 ausgestattet, welche im vorliegenden Beispiel aus Stahl sind und in ein entsprechendes T-Profil einer Führungsanordnung eingreifen können. Durch das Drei-Rollen-System kann das Führungselement 19 einem Kurvenverlauf der Führungsanordnung folgen, ohne dass eine Federung der Rollen 20.1, 20.2 und 21 erforderlich wäre. Eine Federung kann dennoch selbstverständlich implementiert sein.

Die Haltekassette 18 verfügt über eine Applikator-Aufnahme 22, welche als Hohlraum dargestellt ist, welcher sich durch die gesamte Dicke der Haltekassette 18 erstreckt und zur Aufnahme eines Tampon-Applikators, wie in der Fig. 3a gezeigt, ausgerichtet ist. Die Applikator-Aufnahme weist einen runden Querschnitt auf, der durch drei Haltebeulen 17.1, 17.2, 17.3 eingebuchtet ist. Es wurde überraschend gefunden, dass bereits der Kontakt durch diese drei Haltebeulen eine optimale Halterung des Tampon-Applikators in der Führungseinheit ermöglicht, welcher besonders schonend zum Applikator-Material ist. Die Haltekassette 18 kann einstückig ausgebildet sein. Zwischen der Haltekassette 18 und dem Führungselement 19 sorgt eine Rückstellfeder 23 für eine Rückstellkraft, welche eine Bewegung der Haltekassette 18 in der Frontalebene kompensiert.

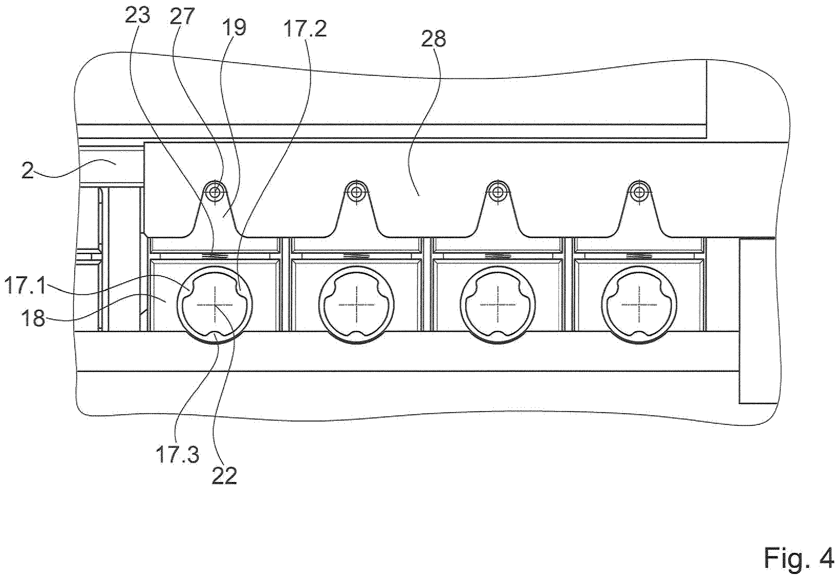

In der Fig. 4 ist ein weiterer Aspekt dargestellt. Die Führungseinheiten 10 können einzeln jeweils über einen Mitnehmer von den Antriebseinheiten in der Förderrichtung bewegt werden. Alternativ oder als Ergänzung kann eine abgeteilte Menge an Führungseinheiten über einen Mitnehmer 28 mit einem eingekerbten Profil ausgestaltet sein, welches mit einem Stift 27 jeweils einer Führungseinheit in Wirkverbindung treten kann und dabei eine Ausrichtung der Führungseinheiten und eine Beförderung derselben ermöglichen. Die Führungseinheiten sind in der vorliegenden Darstellung im Vergleich zu den Fig. 3a, 3b von ihrer Rückseite her gezeigt, aber ebenso erkennbar ist die Haltekassette 18 mit den drei Haltebeulen 17.1, 17.2 und 17.3. Auf der entgegengesetzten Seite des Stiftes 27, welches am Führungselement 19 ausgebildet ist, ist die Führungsanordnung 2 abgebildet, in welche die Rollen (in dieser Darstellung nicht gezeigt) eingreifen können. Diese Art von Mitnehmer 28 eignet sich z.B. in Bearbeitungsbereichen, so z.B. in einer Eingangsstelle, Ausgangsstelle, Heizelement, Formungselement und/oder Kühlelement. Zusätzlich zum Transport der Führungseinheiten kann ein derart gestalteter Mitnehmer 28 für eine Ausrichtung der Führungseinheiten bezüglich eines Bearbeitungselements sorgen.

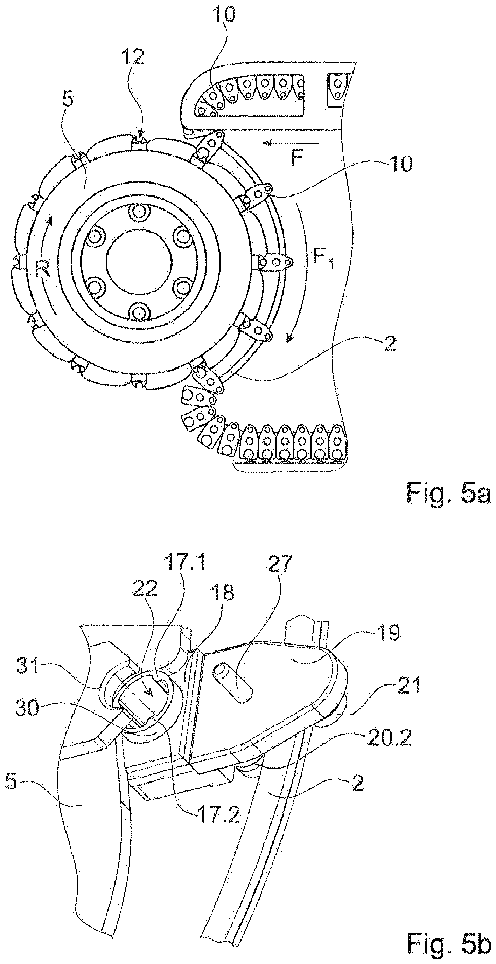

In der Fig. 5a ist eine Ausführungsform des Bearbeitungsbereichs einer Bestückungseinheit 5 dargestellt. Die Bestückungseinheit 5 ist, wie eingangs geschildert, trommelförmig ausgebildet und verfügt über radial angeordnete Bestückungsmitnehmer 12, welche, sobald eine Führungseinheit 10 in den Wirkbereich durch ihre Förderung in Förderrichtung F der Bestückungseinheit 5 gelangen, mit einem Mitnahmeansatz 24 in Wirkverbindung treten können und die Führungseinheiten 10 in den Bearbeitungsradius F1 der Bestückungseinheit 5 übernehmen. Im Verlauf dieses Transports über den Bearbeitungsradius F1, vorzugsweise an einem Zeitpunkt, an dem der Tampon-Applikator konzentrisch zu einer Tampon-Aufnahme der Bestückungseinheit 5 ausgerichtet ist, findet die Bestückung des Tampon-Applikators statt. Im vorliegenden Beispiel ist auch gezeigt, wie eine Mehrzahl an Führungselementen für eine kontinuierliche Bearbeitung und Bestückung sorgen kann.

In der Fig. 5b ist eine Rückansicht des Bestückungsradius F1 mit einer Führungseinheit 10 gezeigt, welche bereits von einem Bestückungsmitnehmer 12 erfasst wurde. Die Führungsanordnung 2 beschreibt an dieser Stelle einen Radius, welcher im Wesentlichen dem Umfang der Bestückungseinheit 5 folgt. Die Führungsanordnung 2 weist eine Schiene auf, in welche die Rollen 20.2, 21 am Führungselement 19 eingreifen. Auf der Rückseite des Führungselements 19, den Rollen abgewandt, befindet sich ein Stift 27, welcher z.B. zur Mitnahme der Führungseinheit 10 durch die Antriebselemente (in diesem Beispiel nicht gezeigt), oder durch abgeteilten Mengentransport über den Mitnehmer (wie oben geschildert) geeignet ist. Die Haltekassette 18 weist eine Applikator-Aufnahme 22 auf, welche über drei Haltebeulen 17.1, 17.2, 17.3 einen Tampon-Applikator halten. Eine Applikator-Aufnahmeblende 30 fluchtet mit einer Tampon-Führung 31 in diesen Bearbeitungsraum, welcher bei einer konzentrischen Anordnung der Längsachsen eine Übertragung des Tampons auf den Tampon-Applikator ermöglicht. Im vorliegenden Beispiel würde der Tampon-Applikator mit seinem distalen Ende nach vorne in die Betrachterebene zeigen und über die Applikator-Aufnahmeblende 30 und die Tampon-Führung 31 von der Bestückungseinheit 5 bestückt werden.

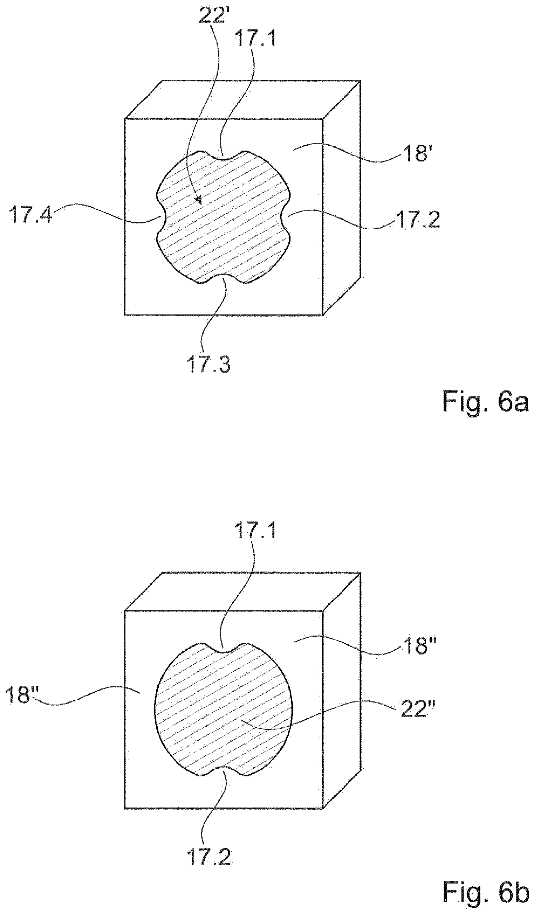

In den Fig. 6a und 6b sind alternative Ausführungsformen für die Haltekassette 18 gezeigt. Die Fig. 6a zeigt eine Haltekassette 18', bei der eine Ausnehmung 22' durch vier Haltebeulen 17.1, 17.2, 17.3 und 17.4 begrenzt ist. Dabei sind die Haltebeulen so ausgerichtet, dass sie im Wesentlichen symmetrisch angeordnet sind. Auch diese Anordnung ermöglicht ein Halten eines Tampon-Applikators, welches schonend ist und gleichzeitig eine sichere Bearbeitung ermöglicht.

Die Fig. 6b zeigt eine weitere alternative Ausführungsform einer Haltekassette 18". Die Haltekassette 18" hat ebenfalls eine Applikator-Aufnahme 22", welche in diesem Fall von zwei Haltebeulen 17.1, 17.2, welche spiegelsymmetrisch zueinander angeordnet sind, begrenzt ist.

Für einen Fachmann ergeben sich aus den gezeigten Ausführungsbeispielen und aus der allgemeinen Offenbarung weitere vorteilhafte Kombinationen, welche hier nicht im Detail beschrieben sind.

Mit der Vorrichtung, der Führungseinheit und dem entsprechenden Verfahren wird ebensolches bereitgestellt, welches sicher einfach zu bedienen und gleichzeitig sehr hohe Prozessgeschwindigkeiten ermöglicht. Zusätzliche Vorteile sind die einfache Wartbarkeit und die Modularität, welche einzelne Bauelemente anders anordnen können als hier im konkreten Beispiel gezeigt.

### Bezugszeichenliste

- 1: Vorrichtung
- 2: Führungsanordnung
- 3: Eingangsstelle
- 4: Ausgangsstelle
- 5: Bestückungseinheit
- 6: Heizelement
- 7: Formungselement
- 8: Kühlelement
- 9: Antriebselement
- 10: Führungseinheit
- 11: Ausrichtradius
- 12: Bestückungsmitnehmer
- 13: Tamponaufnahme
- 14: Anschlagsbereich
- 15: Vereinzelungseinheit
- 16: Stopper
- 17.1 «1.: Haltebeule»
- 17.2 «2.: Haltebeule»
- 17.3 «3.: Haltebeule»
- 18: Haltekassette
- 19: Führungselement
- 20.1: erste Rolle
- 20.2: zweite Rolle
- 21: Gegenrolle
- 22: Applikator-Aufnahme
- 23: Rückstellfeder
- 24: Mitnahmeansatz
- 25: Rastnasen
- 26: Rückstellbereich
- 27: Stift
- 28: Mitnehmer
- 29: Pufferbereich
- 30: Applikator-Aufnahme-Blende
- 31: Tamponführung

- 40: Tamponkörper
- 41: Tampon
- 42: bestückter Tampon-Applikator
- 43: Stössel
- 44: Rückholfaden
- 45: Kopföffnung
- 46: distales Ende
- 47: proximales Ende
- 48: Griffbereich

- F: Förderrichtung
- F1: angepasster Radius
- R: Rotationsrichtung

## Patentansprüche

1. Haltekassette (18; 18'; 18") zur Aufnahme eines Tampon-Applikators, umfassend eine Applikator-Aufnahme (22, 22', 22"), die mindestens eine, insbesondere eine bis vier, Haltebeulen (17.1, 17.2, 17.3, 17.4) aufweist, welche sich dergestalt in eine Ausnehmung der Applikator-Aufnahme (22, 22', 22") erstrecken, dass sie mit einem eingeführten Tampon-Applikator eine form- und/oder kraftschlüssige Verbindung einzugehen vermögen.

2. Haltekassette (18; 18'; 18") gemäss Anspruch 16 zur Verwendung für den Transport von Tampon-Applikatoren in einer Maschineninstallation.

3. Haltekassette (18; 18'; 18") gemäss Anspruch 17 zur Verwendung mit einer Führungseinheit (10) zum Transport von Tampon-Applikatoren auf einer umlaufenden Führungsanordnung (2) mit einer Eingangsstelle (3), an der Tampon-Applikatoren (40) in die Vorrichtung eingespeist werden, und mit einer Ausgangsstelle (4), an der bestückte Tampon-Applikatoren (42) ausgeschieden werden; einer Bestückungseinheit (5), angeordnet zwischen der Eingangsstelle (3) und der Ausgangsstelle (4), und ausgestaltet, um Tampon-Applikatoren (40) mit Tampons (41) zu bestücken und einer Mehrzahl von auf der Führungsanordnung (2) geführter Führungseinheiten (10) zum Transport der Tampon-Applikatoren (40) auf der Führungsanordnung (2).

## Claims

1. Holding cassette (18; 18'; 18") for receiving a tampon applicator, comprising an applicator receptacle (22, 22', 22") which comprises at least one, in particular one to four, holding bulges (17.1, 17.2, 17.3, 17.4) which extend into a recess of the applicator receptacle (22, 22', 22") such that they are capable of forming a positive-fitting and/or force-fitting connection with an inserted tampon applicator.

2. Holding cassette (18; 18'; 18") according to claim 16 for use in the transportation of tampon applicators in a machine installation.

3. Holding cassette (18; 18'; 18") according to claim 17 for use with a guide unit (10) for transporting tampon applicators on an encircling guide arrangement (2) with an entry point (3), at which tampon applicators (40) are fed into the apparatus, and with an exit point (4), at which equipped tampon applicators (42) are discharged; an equipping unit (5), arranged between the entry point (3) and the exit point (4), and designed for equipping tampon applicators (40) with tampons (41), and a multiplicity of guide units (10) which are guided on the guide arrangement (2) for transporting the tampon applicators (40) on the guide arrangement (2).

## Revendications

1. Cassette de maintien (18 ; 18'; 18") destinée à recevoir un applicateur de tampon, comprenant un logement d'applicateur (22, 22', 22") qui présente au moins une, en particulier jusqu'à quatre creux de maintien (17.1, 17.2, 17.3, 17.4) qui s'étendent dans un évidement du logement d'applicateur (22, 22', 22") de façon à pouvoir réaliser une liaison par complémentarité de forme et/ou par adhérence avec un applicateur de tampon introduit.

2. Cassette de maintien (18 ; 18' ; 18") selon la revendication 1, destinée à être utilisée pour le transport d'applicateurs de tampon dans une installation de machine.

3. Cassette de maintien (18 ; 18' ; 18") selon la revendication 2, destinée à être utilisée avec une unité de guidage (10) pour transporter des applicateurs de tampon sur un agencement de guidage (2) périphérique, muni d'un point d'entrée (3) auquel des applicateurs de tampon (40) sont alimentés dans le dispositif, et d'un point de sortie (4) auquel des applicateurs de tampon (42) équipés sont éjectés ; une unité d'équipement (5) qui est disposée entre le point d'entrée (3) et le point de sortie (4) et qui est configurée pour équiper en tampons (41) des applicateurs de tampon (40), et une pluralité d'unités de guidage (10) guidées sur l'agencement de guidage (2) pour transporter les applicateurs de tampon (40) sur l'agencement de guidage (2).
